# EUROPÄISCHE PATENTSCHRIFT

(11) **EP 1 509 623 B1**
(45) Veröffentlichungstag und Bekanntmachung des Hinweises auf die Patenterteilung: **22.07.2009**
(21) Anmeldenummer: 03755950.7
(22) Anmeldetag: 27.05.2003
(51) Int. Cl.: C12Q 1/68, C12P 19/34

(54) **VERMEHRUNG VON RIBONUKLEINSÄUREN**
AMPLIFICATION OF RIBONUCLEIC ACIDS
AMPLIFICATION D'ACIDES RIBONUCLÉIQUES

(30) Priorität: 31.05.2002 DE 10224200
(43) Veröffentlichungstag der Anmeldung: 02.03.2005
(73) Patentinhaber: AmpTec GmbH, 22767 Hamburg (DE)
(72) Erfinder: SCHEINERT, Peter, 22589 Hamburg (DE); KRUPP, Guido, 24622 Gnutz (DE)
(74) Vertreter: von Menges, Albrecht
(86) Internationale Anmeldenummer: PCT/EP2003/005579
(87) Internationale Veröffentlichungsnummer: WO 2003/102232

(56) Entgegenhaltungen:
- EP-A- 1 275 734
- WO-A-00/18966
- US-A- 5 972 607
- US-A- 6 132 997
- US-B1- 6 361 949
- US-B1- 6 379 932

### SEQUENZPROTOKOLL

<110> Artus Gesellschaft für molekularbiologische Diagnostik und Entwicklung mbH
<120> Vermehrung von Ribonukleinsäuren
<130> P 60658
<160> 6
<170> PatentIn version 3.1
<210> 1
   <211> 27
   <212> DNA
   <213> künstliche Sequenz
<220>
   <221> misc feature
   <222> (22)..(24)
   <223> n = a or g or c or t
<400> 1
   gcatcataca agcttggtac cnnntct 27
<210> 2
   <211> 28
   <212> DNA
   <213> künstliche Sequenz
<220>
   <221> misc_feature
   <222> (22)..(25)
   <223> n = a or g or c or t
<400> 2
   gcatcataca agcttggtac cnnnntct 28
<210> 3
   <211> 29
   <212> DNA
   <213> künstliche Sequenz
<220>
   <221> misc feature
   <222> (22)..(26)
   <223> n = a or g or c or t
<400> 3
   gcatcataca agcttggtac cnnnnntct 29
<210> 4
   <211> 30
   <212> DNA
   <213> künstliche Sequenz
<220>
   <221> misc feature
   <222> (22)..(27)
   <223> n = a or g or c or t
<400> 4
   gcatcataca agcttggtac cnnnnnntct 30
<210> 5
   <211> 40
   <212> DNA
   <213> künstliche Sequenz
<220>
   <221> misc feature
   <222> (20)
   <223> Transkriptionsstart
<220>
   <221> misc feature
   <222> (40)
   <223> v = a or g or c
<400> 5
   actaatacga ctcactatag gttttttttt tttttttttv 40
<210> 6
   <211> 21
   <212> DNA
   <213> künstliche Sequenz
<400> 6
   gcatcataca agcttggtac c 21

## Beschreibung

Die vorliegende Erfindung betrifr Verfahren zur Vermehrung von Ribonukleinsäuren, Schritte umfassend, bei denen man
(a) mittels eines Einzelstrang-Primers definierter Sequenz, einer RNA-abhängigen DNA-Polymerase und Desoxyribonukleosidtriphosphaten einen DNA-Einzelstrang durch reverse Transkription aus einer RNA erzeugt;
(b) die RNA entfernt;
(c) mittels eines Einzelstrang-Primers, der eine Box-Sequenz umfaßt, einer DNA-Polymerase und Desoxyribonukleosidtriphosphaten einen DNA-Doppelstrang erzeugt;
(d) den Doppelstrang in Einzelstränge auftrennt;
(e) mittels eines Einzelstrang-Primers, der im 5'-Bereich die Sequenz eines Promotors und im 3'-Bereich dieselbe definierte Sequenz wie der Primer in (a) umfaßt, einer DNA-Polymerase und Desoxyribonukleosidtriphosphaten DNA-Doppelstränge aus einem der in (d) entstandenen Einzelsträngen erzeugt;
(f) mittels einer RNA-Polymerase und Ribonukleosidtriphosphaten eine Vielzahl von RNA-Einzelsträngen erzeugt, die an beiden Enden definierte Sequenzen umfassen.

Die vorliegende Erfindung betrifft ferner Kits, welche für die Durchführung der erfindungsgemäßen Verfahren notwendigen Bestandteile umfassen.

Im Stand der Technik sind eine Vielzahl von Verfahren zur Vermehrung von Nukleinsäuren bekannt. Das bekannteste Verfahren ist die Polymerase-Ketten-Reaktion ("polymerase chain reaction" oder PCR), welche Mitte der 80er Jahre von Kary Mullis entwikelt wurde (vgl. Saiki et al., Science, Vol. 230 (1985), 1350-1354; und EP 201 184).

Bei der PCR-Reaktion lagern sich Einzelstrang-Primer (Oligonukleotide mit einer Kettenlänge von üblicherweise 12 bis 24 Nukleotiden) an eine komplementäre, einzelsträngige DNA-Sequenz an. Die Primer werden mittels einer DNA-Polymerase und den Desoxyribonukleosidtriphosphaten (dNTPs, nämlich dATP, dCTP, dGTP, dTTP) zu einem Doppelstrang verlängert. Der Doppelstrang wird durch Hitzeeinwirkung in Einzelstränge aufgetrennt. Die Temperatur wird so weit gesenkt, dass sich erneut Einzelstrang-Primer an die DNA-Einzelstränge anlagern. Die Primer werden durch die DNA-Polymerase erneut zu einem Zweitstrang elongiert.

Durch Wiederholung der obigen Schritte ist eine exponentielle Vermehrung der Ausgangs-DNA-Stränge möglich, da die Reaktionsbedingungen so gewählt werden können, dass aus nahezu jedem DNA-Einzelstrang bei jedem Reaktionsdurchlauf ein Doppelstrang gebildet wird, der nachfolgend wieder in zwei Einzelstränge aufgespalten wird, welche wiederum als Matrize für weitere Stränge dienen.

Nach Vorschaltung einer reversen Transkription, bei der mittels einer RNA-abhängigen DNA-Polymerase ein DNA-Einzelstrang (die sogenannte cDNA) aus einer mRNA gebildet wird, ist die PCR-Reaktion auch unmittelbar auf die Vermehrung von Nukleinsäuren ausgehend von einer RNA-Sequenz anwendbar (vgl. EP 201 184).

Zu diesem Reaktions-Grundschema wurden in der Zwischenzeit eine Vielzahl von Alternativen entwickelt, die sich in Abhängigkeit des Ausgangsmaterials (RNA, DNA, Einzelstränge, Doppelstränge) und des Reaktionsproduktes (Vermehrung spezifischer RNA- oder DNA-Sequenzen in einer Probe oder Vermehrung aller Sequenzen) voneinander unterscheiden.

In den letzten Jahren werden zunehmend sogenannte Microarrays, zur Analyse von Nukleinsäuren verwendet. Dabei handelt es sich um Trägerplatten auf denen eine Vielzahl unterschiedlicher Nukleinsäure-Sequenzen (meist DNA) in verschiedenen Bereichen gebunden wurden. Üblicherweise wird in einem bestimmten sehr kleinen Bereich lediglich die DNA einer bestimmten Sequenz gebunden, wobei ein Microarray bis zu mehrere 1000 verschiedene Bereiche aufweisen und Sequenzen binden kann.

Werden diese Microarrays mit einer Vielzahl von verschiedenen Nukleinsäure-Sequenzen (meist ebenfalls DNA) aus einer zu untersuchenden Probe unter geeigneten Bedingungen (Salzgehalt, Temperatur, etc.) in Kontakt gebracht, so bilden sich komplementäre Hybride aus den in der Probe befindlichen und den auf der Platte gebundenen Sequenzen. Nicht-komplementäre Sequenzen können abgewaschen werden. Die Bereiche auf dem Microarray, welche DNA-Doppelstränge enthalten, werden ermittelt und ermöglichen einen Rückschluß auf die Sequenz und Menge der Nukleinsäure in der Ausgangsprobe.

Microarrays werden beispielsweise in entsprechenden Verfahren zur Analyse des Expressionsprofils von Zellen, also der Analyse der Gesamtheit der von bestimmten Zellen exprimierten mRNA-Sequenzen, eingesetzt (vgl. Lockhart et al., Nat. Biotechnol. 14 (1996), 1675-1680).

Da die Menge der für diese Analyse zur Verfügung stehenden mRNA üblicherweise beschränkt ist, sind speziell Verfahren zur Vermehrung von Ribonukleinsäuren entwickelt worden, die anschließend mittels Microarrays analysiert werden sollen. Dafür werden die Ribonukleinsäwen gegebenenfalls durch reverse Transkription in die stabilere cDNA-Form überführt.

Verfahren, die eine hohe Amplifikation einer RNA-Population einzelner Zellen ermöglichen sollen, werden beispielsweise in US 5,514,545 beschrieben. Bei diesem Verfahren wird ein Primer verwendet, der eine Oligo-dT-Sequenz und eine T7-Promotor-Region aufweist. Die Oligo-dT-Sequenz lagert sich an die 3'-Poly-A-Sequenz der mRNA an und initiiert so die reverse Transkription der mRNA. Nach alkalischer Denaturierung des RNA/DNA-Heteroduplex wird der zweite DNA-Strang unter Ausnutzung der Haarnadel-Struktur am 3'-Ende der cDNA als Primer gebildet und mittels Nuklease S1 zum linearen Doppelstrang geöffnet. Der DNA-Doppelstrang dient anschließend als Matrize für die T7-RNA-Polymerase. Die so erhaltene RNA dient wiederum als Primer für cDNA-Synthese, wobei hexamere Oligonukleotide beliebiger Sequenz (sog. "random primer") verwendet werden. Der zweite DNA-Strang wird nach Hitzedenaturierung unter Verwendung der genannten T-7-Oligo(dT)-Primer erzeugt. Diese DNA kann erneut als Matrize für die T7 RNA-Polymerase dienen.

Alternativ dazu offenbart US 5,5.45,522 dass auch ein einziges Primer-Oligonukleotid verwendet werden kann, um eine hohe Vermehrungsrate zu erzielen. Dafür wird cDNA aus einer RNA durch reverse Transkription erzeugt, wobei ein Primer verwendet wird, der folgdende Elemente aufweist: (a) 5'-dN₂₀, also beliebige Sequenz über eine Länge von 20 Nukleotiden; (b) minimaler T7-Promotor; (c) Transkriptionsstartsequenz GGGCG; und (d) Oligo-dT₁₅. Die Sythese des zweiten DNA-Stranges wird nach partiellem Verdau der RNA mittels RNase H durchgeführt, indem verbleibende RNA-Oligonukleotide als Primer für Polymerase I dienen. Die Enden der so erhaltenen DNA werden mit T4-DNA Polymerase geglättet.

Ein ähnliches Verfahren wird in US 5,932,451 offenbart, bei dem zusätzlich im 5'-proximalen Bereich noch zwei Box-Primersequenzen addiert werden, die ein doppeltes Immobilisieren via Biotin-Boxprimer ermöglichen.

US 6,132,997 offenbart Verfahren zur Vermehrung von Ribonukleinsäuren, bei denen eine cDNA aus mRNA gewonnen wird und die cDNA unmittelbar für die Transkription verwendet wird. Auch die US 5,972,607 und die US 6,379,932 beschreiben Verfahren zur Vermehrung von Ribonukleinsäuren.

Die genannten Verfahren zur Vermehrung von Ribonukleinsäuren weisen jedoch Nachteile auf. So erzeugen die Verfahren Populationen von Ribonukleinsäuren, die in ihrer Zusammensetzung nicht der Ursprungs-Population entsprechen. Ein Grund dafür besteht in der Verwendung der T7-Promotor-Oligo-dT-Primer, welche primär Ribonukleinsäure-Sequenzen aus dem 3'-Bereich der mRNA amplifizieren. Ferner wurde experimentell belegt, dass die in den genannten Verfahren verwendeten, sehr langen Primer (mehr als 60 Nukleotide) die Bildung von Primer-Primer-Hybriden und eine unspezifische Amplifikation des Primers ermöglichen (Baugh et al., Nucleic Acids Res., 29 (2001), e29). Die bekannten Verfahren erzeugen somit zu nicht unerheblichem Anteil Artefakte, die bei der weiteren Analyse der Nukleinsäuren stören.

Der vorliegenden Erfindung lag somit die Aufgabe zugrunde, ein Verfahren zur Vermehrung von Ribonukleinsäuren zur Verfügung zu stellen, das eine gleichmäßige und vor allem eine gut reproduzierbare Vermehrung der Ribonukleinsäuren im Ausgangsmaterial ermöglicht.

Diese Aufgabe wurde nunmehr durch Verfahren zur Vermehrung von Ribonukleinsäuren gelöst, welche Schritte umfassen, bei denen man
(a) mittels eines Einzelstrang-Primers definierter Sequenz, einer RNA-abhängigen DNA-Polymerase und Desoxyribonukleosidtriphosphaten einen DNA-Einzelstrang durch reverse Transkription aus einer RNA erzeugt;
(b) die RNA entfernt;
(c) mittels eines Einzelstrang-Primers, einer DNA-Polymerase und Desoxyribonukleosidtriphosphaten einen DNA-Doppelstrang erzeugt, wobei der Einzelstrang-Primer eine Box-Sequenz, eine Sequenz von 3 bis 6 beliebigen Nukleotiden sowie eine ausgewählte Trinukleotidsequenz umfaßt, wobei sich die Box-Sequenz im 5'-Bereich des Einzelstrang-Primers befindet und eine Sequenz mit einer Länge von 10-25 Nukleotiden umfaßt, welche eine geringe Homologie zu Gensequenzen aufweist, welche von den Organismen exprimiert werden, aus denen die zu vermehrende RNA gewonnen wurde;
(d) den Doppelstrang in Einzelstränge auftrennt;
(e) mittels eines Einzelstrang-Primers, der im 5'-Bereich die Sequenz eines Promotors und im 3'-Bereich dieselbe definierte Sequenz wie der Primer in (a) umfaßt, einer DNA-Polymerase und Desoxyribonukleosidtriphosphaten DNA-Doppelstränge aus einem der in (d) entstandenen Einzelsträngen erzeugt;
(f) mittels einer RNA-Polymerase und Ribonukleosidtriphosphaten eine Vielzahl von RNA-Einzelsträngen erzeugt, die an beiden Enden definierte Sequenzen umfassen.

Erfindungsgemäß wurde überraschenderweise festgestellt, dass die genannte Kombination von Verfahrensschritten zu einer besonders gleichmäßigen Amplifikation der im Ausgangsmaterial befindlichen Ribonukleinsäuren führt und gleichzeitig die Entstehung von Artefakten verhindert. Das erfindungsgemäße Verfahren stellt somit eine Verbesserung der Verfahren zur Vermehrung von Nukleinsäuren dar und ermöglicht eine Verbesserung der Verfahren zur Analyse von Ribonukleinsäuren mittels Microarrays.

Gemäß dem beschriebenen Verfahren werden bei der Vermehrung RNA-Einzelstränge erzeugt, welche die inverse Sinnrichtung (antisense Sequenz) wie das RNA-Ausgangsmaterial aufweisen.

Gemäß einer Ausführungsform der Erfindung wurde die als Matrize in (a) dienende RNA aus Zellen eines Organismus isoliert. Die Isolierung von mRNA aus Zellen eines vielzelligen Organismus ist besonders bevorzugt ist.

Der in (a) verwendete Einzelstrang-Primer ist ein Primer mit einer beliebigen definierten Sequenz, d.h. er enthält keine zufällige Abfolge von Nukleotiden. Es können auch mehr als ein definierter Primer zum Einsatz gelangen.

Der in (a) verwendete Primer umfaßt vorzugsweise eine Oligo-dT-Sequenz, also eine Sequenz, in der mehrere dT-Nukleotide aneiander gereiht sind. Dies weist den Vorteil auf, dass die Anlagerung des

Primers im Bereich der poly-A-Sequenz der mRNA erfolgt. Es wird somit in der reversen Transkription nahezu ausschließlich mRNA transkribiert.

Erfindungsgemäß ist es besonders bevorzugt, das in (a) ein 5'-(dT)₁₈V-Primer für die reverse Transkription verwendet wird. Darunter wird ein Primer verstanden, der 18 dT-Desoxyribonukleotid-Monomere gefolgt von einem einzelnen Desoxyribonukleotid-Monomer anderer Art (also dA, dC oder dG, hier als V bezeichnet) aufweist. Dieser Primer ermöglicht eine reverse Transkription, die nahezu ausschließlich Sequenzen transkribiert, welche im unmittelbaren 5'-Bereich der Poly-A-Seqenz beginnen. Die Verwendung dieses Primers unterdrückt somit die Bildung von Transkriptions-Artefakten, welche durch Anlagerung der üblichen Oligo-dT-Primer in größeren Poly-A-Bereichen der mRNA bei den im Stand der Technik bekannten Verfahren entstehen.

Alternativ dazu kann aber in (a) auch ein Primer verwendet werden, der eine Homologie zu einer oder mehreren bestimmten Sequenz(en) in der Probe aufweist. Bei entsprechenden Verfahren ist die Vermehrung der Ribonukleinsäuren somit auf bestimmte Zielsequenzen beschränkt.

Erfindungsgemäß ist es bevozugt die RNA in den DNA-RNA-Hybriden in (b) durch RNase aufzuspalten. Dabei können beliebige RNasen verwendet werden. Die Verwendung von RNase I und/oder RNase H ist bevorzugt. Dadurch werden ferner alle RNAs eliminiert, die beim ersten Schritt nicht in cDNA umgeschrieben wurden, insbesondere ribosomale RNAs, aber auch andere zelluläre RNAs, die nicht den für mRNA charakteristischen Poly(A)-Schwanz haben. Die durch reverse Transkription entstandenen DNA-RNA-Hybride können auch durch Hitzeeinwirkung in die Einzelstrang-Form überführt werden. Die Verwendung der RNasen weist den Vorteil auf, dass die üblicherweise ebenfalls in der Probe vorliegende genomische DNA nicht linearisiert wird und somit auch nicht als Hybridisierungspartner für die Primer in den nachfolgenden Schritten zur Verfügung steht. Besondere Vorteile ergeben sich durch die Verwendung der RNase I, da diese leicht durch Hitze inaktiviert werden kann. Das erfindungsgemäße Verfahren hat gerade die Vermehrung der Ribonukleinsäuren zum Ziel; eine stabile RNase könnte diesem Ziel entgegen wirken und müsste umständlich aus dem Reaktionsansatz entfernt werden.

In (c) wird ein Einzelstrang-Primer verwendet, der eine Box-Sequenz umfaßt. Im Rahmen der vorliegenden Erfindung wird eine RNA- oder DNA-Sequenz als Box-Sequenz bezeichnet, wenn diese eine definierte Sequenz mit einer Länge von 10 bis 25 Nukleotiden aufweist, die nur eine geringe Homologie zu Gensequenzen aufweist, welche von den Organismen exprimiert werden, von denen die zu vermehrende Ribonukleinsäure gewonnen wurde.

Die geringe Homologie einer potentiellen Box-Sequenz zu entsprechenden Gensequenzen kann experimentell durch eine Standard Northem Analyse bestimmt werden. Dafür können RNA-Proben des Organismus, von dem die zu vermehrenden Ribonukleinsäuren gewonnen wurden (bspw. Pflanze, Mensch, Tier), elektrophoretisch aufgetrennt und auf eine Membran übertragen werden. Eine geringe Homologie liegt dann vor, wenn eine Hybridisierung mit einem markierten Oligonukleotid, das die Box-Sequenz aufweist, unter stringenten Hybridisierungsbedingungen kein Hybridisierungsignal erzeugt. Stringente Bedingungen können beispielsweise durch Waschen nach der Hybridisierung mit 0,1 * SSC, 0,1% SDS für 40 Minuten bei 25 Grad Celsius realisiert werden.

Alternativ zu der experimentellen Verifizierung der Box-Sequenz kann die Ermittlung einer Sequenz, die nur eine geringe Homologie zu bekannten Gensequenzen aufweist, welche in Vielzellern exprimiert werden, über Datenbanken erfolgen. Im Stand der Technik sind die bekannten Gensequenzen, welche in Vielzellern exprimiert werden in Form von Datenbanken allgemein zugänglich; entweder als Gensequenzen mit bekannter Funktion oder - falls diese unbekannt ist - in Form von sogenannten "expressed sequence tags" oder ESTs. Eine Sequenz wird dabei als Sequenz bezeichnet, die nur eine geringe Homologie zu bekannten Gensequenzen aufweist, und ist demgemäß als Box-Sequenz geeignet, wenn diese im Vergleich zu allen in einer entsprechenden Datenbank gespeicherten Sequenzen über die gesamte Länge von 10 bis 25 Nukleotiden mindestens 20%, vorzugsweise mindestens 30 oder 40 % Sequenzunterschiede aufweist. Das heißt, es sind mindestens 2 Sequenzunterschiede bei einer Gesamtlänge von 10 Nukleotiden vorhanden, bzw. 4 bei einer Gesamtlänge von 20 Nukleotiden. Die Sequenzidentität, bzw. die Unterschiede zwischen zwei Sequenzen werden dabei vorzugsweise unter Verwendung der BLAST Software ermittelt.

Eine bestimmte Sequenz kann somit im Hinblick auf eine bestimmte Verwendung als Box-Sequenz bezeichnet werden. Soll mRNA eines Menschen mit dem erfindungsgemäßen Verfahren vermehrt werden, so muß die beschriebene, geringe Homologie im Vergleich zu einer humanen Datenbank vorliegen oder die experimentelle Verifizierung der Box-Sequenz gegen humane RNA-Sequenzen über ein Northern-Blot erfolgen. Soll mRNA einer Pflanze mit dem erfindungsgemäßen Verfarhen vermehrt werden, so muß die beschriebene, geringe Homologie im Vergleich zur den Ribonukleinsäuren der Pflanze gegeben sein. Eine Sequenz, die daher für ein bestimmtes erfindungsgemäßes Verfahren als Box-Sequenz infrage kommt, kann in einem anderen Verfahren möglicherweise nicht als Box-Sequenz verwendet werden.

Die Box-Sequenz wird zudem vorzugsweise so gewählt, dass diese keine viralen Sequenzen umfaßt und zwar weder kodierende noch regulatorische Sequenzen (Promotoren oder Terminatoren) von Viren oder Bakteriophagen.

Die Verwendung eines Primers, der eine entsprechende Box-Sequenz umfaßt, in dem erfindungsgemäßen Verfahren bietet besondere Vorteile, da die Entstehung von Amplifikationsartefakten deutlich reduziert wird.

Die Box-Sequenz befindet sich dabei im 5'-Bereich des in (c) genutzten Primers. Der Primer umfaßt ferner vorzugsweise eine Sequenz von 3 bis 6 beliebigen Nukleotiden (N3-N6), sowie eine ausgewählte Trinukleotidsequenz (wie z.B. TCT). Alternativ kann eine Mischung verschiedener Trinukleotidsequenzen Teil des Primers sein.

Der Primer, der die Box-Sequenz umfaßt, weist vorzugsweise eine Länge von 40 Nukleotiden auf, wobei eine Länge von etwa 30 Nukleotiden besonders bevorzugt ist.

Gemäß einer besonders bevorzugten Ausführungsform der Erfindung wird in (c) ein Einzelstrang-Primer verwendet wird, der neben der Box-Sequenz eine besonders geeignete Sequenz von mindestens 6 Nukleotiden umfaßt. Der in (c) verwendete Einzelstrang-Primer kann beispielsweise die folgende Sequenz GCA TCA TAC AAG CTT GGT ACC N₃-₆ TCT (27-30 nt) aufweisen.

Als DNA-Polymerase in (c) und (e) kann eine beliebige DNA-abhängige DNA-Polymerase verwendet werden. Vorzugsweise wird eine Reverse Transkriptase verwendet. Besondere Vorteile des erfindungsgemäßen Verfahrens ergeben sich, wenn man als DNA-Polymerase eine Reverse Transkriptase verwendet, da diese doppelsträngige DNA-Bereiche nicht auflöst. Für die DNA-Polymerisation in (a), (c) und (e) benötigt man ferner vier Desoxyribonukleosidtriphosphate, üblicherweise dATP, dCTP, dGTP und dTTP.

In (d) können die DNA-Doppelstränge durch beliebige Verfahren in Einzelstränge aufgetrennt werden. Vorzugsweise erfolgt die Auftrennung durch Hitzeeinwirkung.

Der in (e) eingesetzte Einzelstrang-Primer umfaßt einen Promotor. Eine Promotor-Sequenz ermöglicht die Bindung der RNA-Polymerase, wodurch die Synthese eines RNA-Stranges initüert wird. Vorzugsweise verwendet man in (e) einen Einzelstrang-Primer, der die Sequenz eines hochspezifischen RNA-Polymerase-Promotors umfaßt, wie T7, T3 oder SP6. Beispielsweise hat ein Primer mit T7-Promotor folgende Sequenz, ACT AAT ACg ACT CAC TAT A g⁺¹ g (dT)₁₈V (40 nt).

Die Auswahl der in dem erfindungsgemäßen Verfahren eingesetzten RNA-Polymerase(n) erfolgt in Abhängigkeit der in dem Primer vorhandenen Promotor-Sequenz. Wurde ein Primer mit Sequenzen der T7-Polymerase verwendet, so wird eine T7-RNA-Polymerase eingesetzt.

Für die Bildung von Ribonukleinsäuren benötigt man ferner Ribonukleosidtriphosphate, üblicherweise werden ATP, CTP, GTP und UTP eingesetzt.

Die erfindungsgemäßen Verfahren ermöglichen erstmals eine starke spezifische Vermehrung der RNA-Ausgangssequenzen, bei der die Gesamtsequenz der RNA-Population repräsentiert wird. Die Vermehrung der Ausgangs-RNA-Sequenz erfolgt vorzugsweise um einen Faktor von mindestens 500, wobei eine Vermehrung um einen Faktor von mehr als 1000 besonders bevorzugt ist.

Von der vorliegenden Erfindung werden schließlich auch Verfahren umfaßt, bei denen man den Einzelstrang-Primer und Primer-verursachte Artefakte (z.B. Dimere) entfernt, bevor die RNA-Polymerase zugegeben wird.

Eine weitere Vermehrung der Ribonukleinsäuren kann in Verfahren erzielt werden, bei denen man im Anschluß an (f):
(g) aus den in (f) erzeugten RNA-Einzelsträngen, einem Einzelstrang-Primer, der die Box-Sequenz umfaßt, einer RNA-abhängigen DNA-Polymerase und Desoxyribonukleosidtriphosphaten wird ein DNA-Einzelstrang durch reverse Transkription erzeugt;
(h) die RNA wird entfernt;
(i) aus den in (h) erzeugten DNA-Einzelsträngen mittels eines Einzelstrang-Primers, der im 5'-Bereich die Sequenz eines Promotors und im 3'-Bereich dieselbe definierte Sequenz, wie der in (a) genutzte Primer umfaßt, einer DNA-Polymerase und Desoxyribonukleosidtriphosphaten DNA-Doppelstränge erzeugt; und
(j) mittels einer RNA-Polymerase und Ribonukleosidtriphosphaten eine Vielzahl von RNA-Einzelsträngen erzeugt.

Durch diese Verfahrensvariante werden besondere Vorteile erzielt. Die definierten Enden der in dem Verfahren gemäß (a) bis (f) erzeugten Ribonukleinsäuren ermöglichen eine reverse Transkription in DNA, die anschließend wiederum als Basis für weitere RNA-Synthese von dem Promotor ausgehend dient. Auf diese Weise läßt sich die Menge der vermehrten Ribonukleinsäure nochmals um einen Faktor von mindestens 50 steigern.

Vorzugsweise wird das Verfahren dabei so durchgeführt, dass der in (i) genutzte Einzelstrang-Primer dieselbe Sequenz wie der in (a) genutzte Primer aufweist.

Der in (g) eingesetzte Primer kann identisch mit dem in (c) benutzten Primer sein. Alternativ dazu umfaßt der in (g) eingesetzte Primer nur die wohldefinierte Box-Sequenz umfassen und nicht die unspezifischeren Elemente des Primers, die für (c) benötigt wurden. Der in (g) eingesetzte Primer kann somit beispielsweise die Sequenz GCA TCA TAC AAG CTT GGT ACC (21 nt) aufweisen.

Die in (h) erzeugte RNA kann mittels beliebiger im Stand der Technik bekannter Verfahren entfernt werden, wobei eine Hydrolyse mittels einer RNase jedoch bevorzugt wird.

Vor der Durchführung der Transkription (Schritte (f) und (j) der erfindungsgemäßen Verfahren) kann es vorteilhaft sein, die Nukleinsäuren nach im Stand der Technik bekannten Verfahren zu reinigen. Dabei sollten in erster Linie im Überschuß eingesetzte Primer und von diesen abgeleitete Artefakte (z.B. Primer-Dimere) abgereinigt werden.

Das oben beschriebene erfindungsgemäße Verfahren erzeugt ausschließlich RNA-Einzelstränge mit inverser Sinnrichtung (sogenannte Antisense-Stränge). Vom Umfang der vorliegenden Erfindung werden jedoch ebenfalls Verfahren erfaßt, die anschließend mittels im Stand der Technik üblicher Verfahren (reverse Transkription, PCR, cDNA-Zweitstrangsynthese, Transkription, etc.) aus dem RNA-Einzelstrang inverser Sinnrichtung einen DNA-Doppelstrang, einen DNA-Einzelstrang beliebiger Sinnrichtung oder einen RNA-Einzelstrang mit Sinnrichting (sogenannter Sense-Strang) erstellen. Die genaue Art des Verfahrensproduktes hängt natürlich in erster Linie von der geplanten Verwendung ab.

Die vorliegende Erfindung betrifft ferner Kits, die alle Reagentien zur Vermehrung von Ribonukleinsäuren mittels des erfindungsgemäßen Verfahrens zur Verfügung stellen. Entsprechende Kits umfassen die folgenden Bestandteile:
(a) mindestens einen Einzelstrang-Primer, der die Sequenz eines Promotors umfaßt;
(b) mindestens einen Einzelstrang-Primer, der eine Box-Sequenz umfaßt;
(c) eine RNA-abhängige DNA-Polymerase;
(d) Desoxyribonukleosidtriphosphate;
(e) eine DNA-abhängige DNA-Polymerase;
(f) eine RNA-Polymerase; und
(g) Ribonukleosidtriphosphate.

Das Kit umfaßt somit mindestens zwei verschiedene Einzelstrang-Primer, welche durch die oben beschriebenen Merkmale gekennzeichnet sind. Das Kit kann jedoch in Abhängigkeit der geplanten Verfahren mehr als zwei Primer und auch weitere Reagenzien umfassen.

Das Kit kann zusätzlich RNase I und/oder RNase H umfassen.

Die in dem Kit vorliegende DNA-Polymerase ist vorzugsweise eine Revese Transkriptase oder eine andere DNA-Polymerase, welche die Eigenschaft hat, doppelsträngige DNA-Breiche nicht aufzulösen.

Das Kit kann ferner eine Zusammenstellung zur Markierung von DNA mit einer nachweisbaren Verbindung und einen oder mehrere DNA-Microarrays umfassen. Mit dem Kit können somit alle Bestandteile zur Durchführung einer Expressionsanalyse angeboten werden. Üblicherweise werden die einzelnen Komponenten in getrennten Behältern verpackt sein; es kann jedoch auch möglich sein, Bestandteile, die in einem Verfahrensschritt verwendet werden, gemeinsam in einen Behälter abzupacken.

Demgemäß betrifft die vorliegende Erfindung ferner Verfahren zur Analyse von Nukleinsäuren, bei denen man eine Ribonukleinsäure gewinnt, mittels eines der erfindungsgemäßen Verfahren vermehrt und unter Verwendung eines Microarrays analysiert. Die Ribonukleinsäure wird üblicherweise durch Isolierung aus einer biologischen Probe gewonnen. Die durch das erfindungsgemäße Verfahren vermehrte Ribonukleinsäure kann vor Analyse mittels Microarrays durch reverse Transkription in cDNA überführt werden. Das Verfahren ermöglicht die Analyse der Menge und/oder Sequenz der cDNA. Auch die in den Zwischenschritten gewonnen DNAs können genutzt werden, bspw. Um durch Klonierung eine repräsentative Genbank zu erhalten, in der die in einer biologischen oder in einer im Labor hergestellten Probe exprimierten Gene enthalten sind.

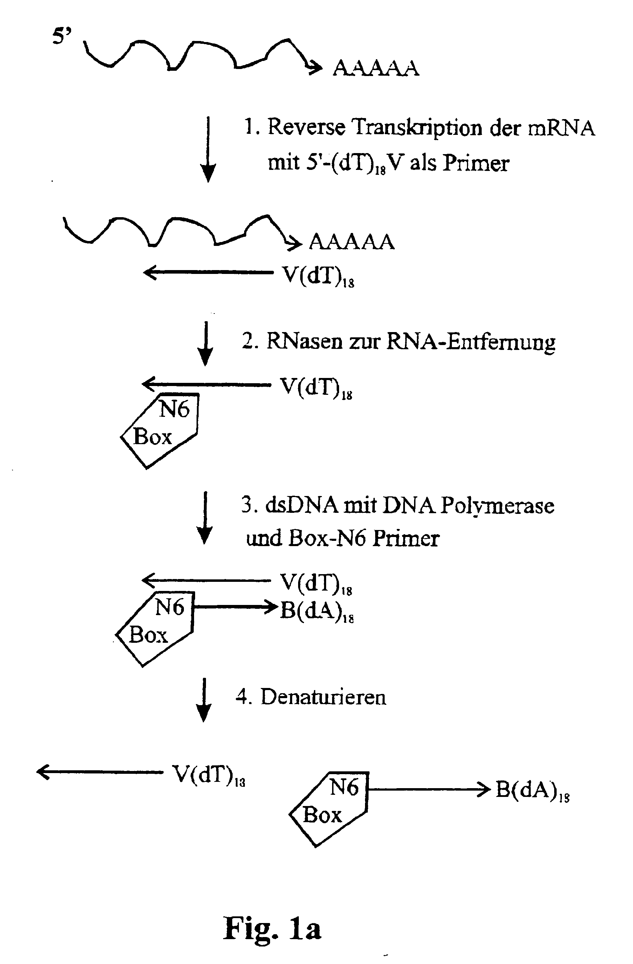

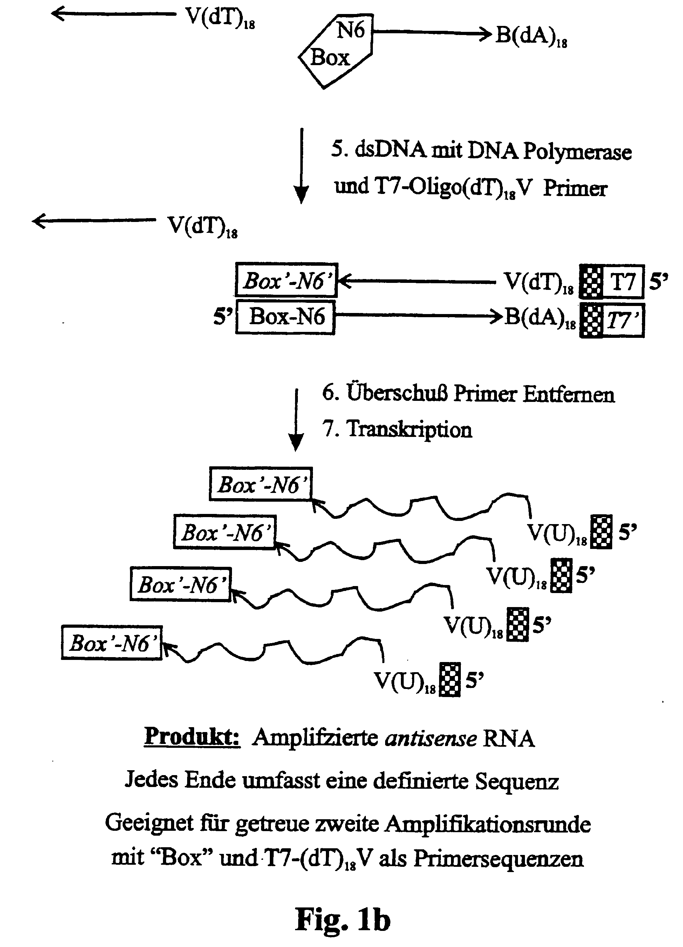

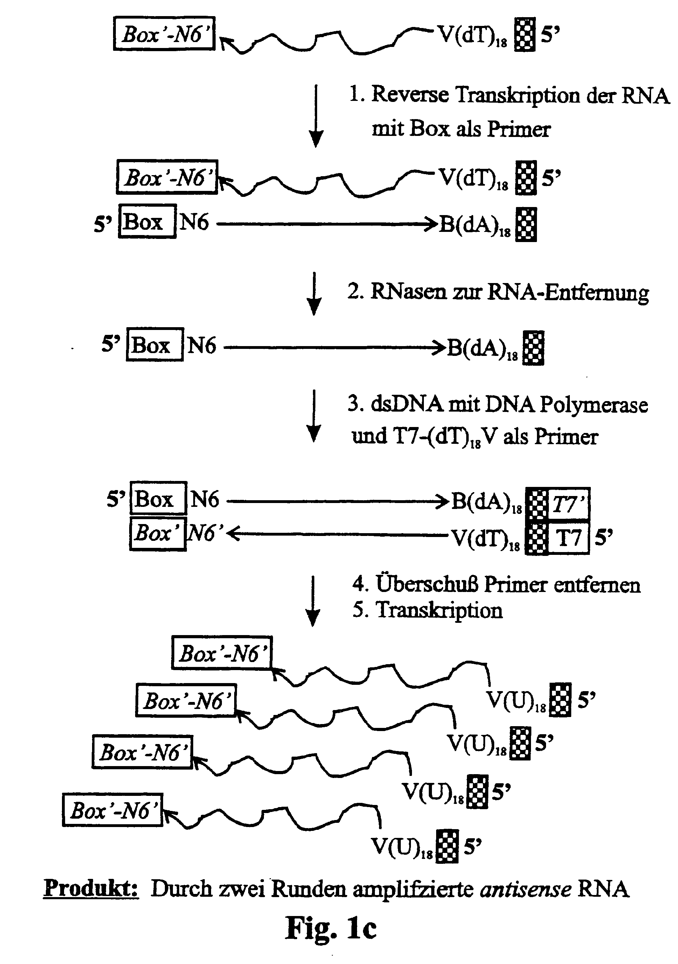

Das erfindungsgemäße Verfahren wird in Fig. 1 beispielhaft dargestellt: Zunächst wird ausgehend von RNA die Synthese eines DNA-Einzelstranges durch reverse Transkription durchgerührt, wobei ein geankerter Oligo(dT)₁₈V-Primer verwendet wird. Dieses Vorgehen ermöglicht es, den Übergang der Poly(A)-Enden der mRNA zum 3'-UTR-Bereich umzuschreiben. Im nächsten Schritt wird die RNA aus dem RNA/cDNA-Heteroduplex mittels RNase H / RNase I eliminiert und die restliche RNA (vor allem ribosomale RNA) durch RNase I entfernt.

Die Synthese des zweiten, komplementären DNA-Stranges wird zur Einführung der Box-Sequenz durch Verwendung eines speziellen Primers genutzt. Der Primer besteht aus einem Teilabschnitt, in dem 3-9 beliebige Nukleotide aneinander gereiht wurden, und einem zweiten Teilabschnitt, der die Box-Sequenz umfaßt.

Nach Anlagerung des Primers erfolgt die Auffüllung zum Doppelstrang mittels der DNA-Polymerase. Überschüssiger Primer wird entfernt und nach Hitzedenaturierung des Doppelstranges wird die Temperatur abgesenkt, wodurch ein Primer hybridisieren kann, der einen T7-Promotor und die Sequenz (dT)₁₈V umfaßt. Ein weiterer DNA-Strang wird durch Elongation des Primers erhalten. Anschließend werden die im Überschuß vorliegenden Primer sowie gegebenenfalls Primer-verursachte Artefakte (z.B. Dimere) entfernt und die Amplifikation der RNA erfolgt per in vitro-Transkription ausgehend von dem T7-Promotor.

In Fig. 1c wird das Verfahren zur Vermehrung von Ribonukleinsäuren mittels der oben beschriebenen Schritte (g) bis (j) gezeigt. Unter Verwendung eines Primers, der die Box-Sequenz umfaßt, einer reversen Transkriptase und den dNTPs wird die in Schritt (f) erzeugte Ribonukleinsäure revers transkribiert. RNasen verdauen den RNA-Strang. Unter Einsatz eines Primers, der einen Promotor und die Oligo-dT Sequenz umfaßt wird ein zweiter DNA-Strang erzeugt, der als Matrize für die RNA-Polymerase in der Transkription dient.

Die Abfolge und detaillierte Durchführung der Reaktionsschritte des erfindungsgemäßen Verfahrens werden nachfolgend beispielhaft dargestellt:

### A. Erste Amplifikationsrunde (vgl. Fig. 1a, 1b)

### A1. Reverse Transkription von 100 ng Gesamt-RNA mit Oligo(dT)₁₈V-Primer:

| Erststrang-DNA-Synthese: | |
|---|---|
| RNA (50 ng/µl): | 2 µl |
| Ollgo(dT)₁₈V (5pmol/µl): | 1,5 µl |
| dNTP-Mix (10 mM): | 1 µl |
| DEPC-H₂O | 3,5 µl |

4 Min bei 65°C in einem Thermocycler **mit Heizdeckel(!)** inkubieren dann auf Eis stellen

| Erststrang-cDNA-Synthesemix | |
|---|---|
| 5 × RT-Puffer | 4 µl |
| RNase-Inhibitor (20 U/µl) | 1 µl |
| Superscript II (200 U/µl) | 1 µl |
| DEPC-H₂O | 6 µl |

Den Erststrang-cDNA-Synthesemix auf Eis zusammen pipettieren und zu den Proben im Eisbad geben. Die Proben anschließend in den auf 42°C vorgewärmten Thermocycler stellen.

### Inkubation der Proben:

37°C/ 5 Minuten
42°C/50 Minuten
45°C/10 Minuten
50°C/10 Minuten
70°C/15 Minuten (zur Inaktivierung des Enzymes)

Anschließend Proben auf Eis stellen.

### A2. Entfernung der RNA

| Entfernung der RNA aus dem Reaktionsansatz | |
|---|---|
| Erststrang-cDNA | 20 µl |
| RNase-Mix (RNase H/RNase I je 5 U/µl) | 1 µl |

Bei 37°C für 20 Minuten inkubieren, anschließend auf Eis stellen. Zur Entfernung der ribosomalen RNA wurde auf die Verwendung von RNase A verzichtet, da dieses Enzym nur sehr schwer zu aktivieren ist. Die verwendete Rnase I kann an dieser Stelle optimal durch Inkubation bei 70°C für 15 Minuten vollständig inaktiviert werden.

### A3. Doppelstrang Template DNA mit Box-random-Primer und mit T7-(dT)₁₈V

| Random Priming der Erststrang cDNA mit Box-random-Primer | |
|---|---|
| Erststrang-cDNA | 21 µl |
| dNTP-Mix (10 mM) | 1 µl |
| Box-random-Primer (10 pmol/µl) | 1 µl |
| 10× Polymerase Puffer | 6 µl |
| H₂O | 20 µl |

Inkubation:
70°C/l Minute
37°C/l Minute
1 µl Reverse Transkriptase (5U/µl) zur Probe geben
37°C/30 Minuten

### Entfernung von Überschuß Primer

1 µl Exonuklease I (10U/µl)
37°C/5 Minute
96°C/6 Minuten

### Anschließend Proben auf Eis stellen.

### Doppelstrang Template DNA mit T7-(dT)₁₈V

2 µl T7-(dT)₁₈V Primer (10pmol/µl)
70°C/l Minute
42°C/l Minute
1 µl Reverse Transkriptase (5U/µl) **zur Probe geben**
42°C/30 Minuten

### Auf 37°C abkühlen

1 µl T4 DNA-Polymerase (10U/µl)
37°C/l Minute
65°C/l Minute

Anschließend Proben auf Eis stellen.

### A4. Reinigung der cDNA mit High-Pure PCR Purification Kit (Roche)

| Reinigung der cDNA | |
|---|---|
| Reaktionsmix | 50µl |
| Binding-buffer | 250 µl |
| Carrier (cot-1-DNA, 100ng/µl) | 3 µl |

Den Mix auf die Säulchen pipettieren und für 1 Minute in einer Tischzentrifuge bei max. Umdrehung zentrifugieren. Den Durchlauf verwerfen, die Säulchen mit 500 µl Waschpuffer auffüllen und wie oben zentrifugieren. Den Durchlauf verwerfen, die Säulchen mit 200 µl Waschpuffer auffüllen und wie oben zentrifugieren.

Die Säulchen in ein neues 1,5 ml-Eppendorf-Gefäß überführen und mit 50 µl Elutions-Puffer auffüllen, eine Minute inkubieren und dann wie oben zentrifugieren. Den Elutionsvorgang einmal wie beschrieben wiederholen.

### A5. Ethanol-Fällung der gereinigten cDNA

Die Pellet Paint™-Carrier-Stocklösung nicht vortexen und immer dunkel aufbewahren. Längere Lagerung bei -20°C, kleinere Aliquots können für ca. 1 Monat bei 4°C gelagert werden.

| Ethanol Fällung | |
|---|---|
| Eluat | 100 µl |
| Carrier (Pellet Paint^{™}) | 2 µl |
| Natrium-Acetat | 10 µl |
| Alkohol abs | 220 µl |

Den Ansatz gut mischen (nicht vortexen) und die cDNA bei Raumtemperatur für 10 Minuten bei maximaler Umdrehung pelletieren. Den Überstand abnehmen und das Pellet einmal mit 200 µl 70% Ethanol waschen 1 Minute wie oben zentrifugieren und den Überstand vollständig mit einer Pipette entfernen. Zum Trocknen des Pellets das Eppendorf-Gefäß für ca. 5 Minuten geöffnet bei Raumtemperatur stehen lassen. **Nicht in der speedvac trocknen!** Anschließend das Pellet in 8 µl Tris-Puffer (pH 8,5) lösen und auf Eis stellen.

### A6. Amplifikation per in vitro-Transkription

| **In vitro Transkription:** | |
|---|---|
| Template DNA | 8 µl |
| ATP/CTP/GTP/UTP (je 75 mM) | 2 µl |
| 10x Puffer | 2 µl |
| T7-RNA-Polymerase | 2 µl |

Alle Reaktionskomponenten auftauen und den Ansatz **bei Raumtemperatur, niemals auf** Eis zusammenpipettieren, da der Spermidin-Anteil im Reaktionspuffer zu einer Präzipitation des Templates führen würde. Für die Reaktion 0,5 ml bzw. 0,2 ml Rnase-frei PCR-Tubes verwenden.
Die Transkription über Nacht bei 37°C in einem Thermocycler mit Heizdeckel oder in einem Hybridisierungsofen inkubieren. 1-2 µl des Ansatzes auf ein natives 1,5%-Agarose-Gel auftragen. Anschließend 1 µl DNase pro Reaktion zugeben und für weitere 15 Minuten bei 37°C inkubieren. Zur Reinigung der RNA das RNeasy-Kit von Qiagen benutzen und nach dem Protokoll zur RNA-clean-up vorgeben. Die RNA anschließend mit 2x50 µl DEPC-Wasser eluieren und wie bei Schritt 6 beschrieben mit Ethanol fallen. Das RNA-Pellet in 5 µl DEPC-Wasser lösen.

Die RNA ist jetzt fertig um eine Markierung für die Microarray-Hybridisierung, bzw. um eine zweite Amplifikationsrunde durchzuführen.

### B. Zweite Amplifikationsrunde (vgl. Fig. 1c)

### B1. Reverse Transkription der amplifizierten RNA mit Box Primer:

| Erststrang-DNA-Synthese: | |
|---|---|
| RNA der 1. Runde: | 4 µl |
| Box Primer (5pmol/µl): | 2 µl |
| dNTP-Mix (10 mM): | 1 µl |
| DEPC-H₂O | 2 µl |

4 Min bei 65°C in einem Thermocycler **mit Heizdeckel(!)** inkubieren dann auf Eis stellen

| Erststrang-cDNA-Synthesemix | |
|---|---|
| 5 × RT-Puffer | 4 µl |
| RNase-Inhibitor (20 U/µl) | 1 µl |
| DEPC-H₂O | 5 µl |

Den Erststrang-cDNA-Synthesemix auf Eis zusammen pipettieren und zu den Proben im Eisbad geben. Die Proben anschließend in den auf 48°C vorgewärmten Thermocycler stellen.

### Inkubation der Proben:

48°C/l Minute
Abkühlen auf 45°C
1 µl Reverse Transkriptase (5U/µl) zur Probe geben
45°C/30 Minuten
70°C/15 Minuten

Anschließend Proben auf Eis stellen.

### B2. Entfernung der RNA

| Entfernung der RNA aus dem Reaktionsansatz | |
|---|---|
| Erststrang-cDNA | 20 µl |
| RNase-Mix (RNase H/RNase I je 5 U/µl) | 1 µl |

Bei 37°C für 20 Minuten inkubieren, anschließend auf Eis stellen. Zur Entfernung der ribosomalen RNA wurde auf die Verwendung von RNase A verzichtet, da dieses Enzym nur sehr schwer zu aktivieren ist. Die verwendete Rnase I kann an dieser Stelle optimal durch Inkubation bei 70°C für 15 Minuten vollständig inaktiviert werden.

### B3. Doppelstrang Template DNA mit T7-(dT)₁₈V Primer

| Template DNA aus Erststrang cDNA mit T7-(dT)₁₈V Primer | |
|---|---|
| Erststrang-cDNA | 21 µl |
| dNTP-Mix (10 mM) | 1 µl |
| T7-(dT)₁₈V Primer (10 pmol/µl) | 2 µl |
| 10× Polymerase Puffer | 6 µl |
| H₂O | 20 µl |

Inkubation:
96°C/l Minute
42°C/l Minute
1 µl Reverse Transkriptase (5U/µl) zur Probe geben
42°C/30 Minuten

Glättung der dsDNA-Enden
Auf 37°C abkühlen
1 µl T4 DNA-Polymerase (10U/µl)
37°C/3 Minuten
96°C/15 Minuten

### Anschließend Proben auf Eis stellen.

### B4. Reinigung der cDNA mit High-Pure PCR Purification Kit (Roche)

| **Reinigung der cDNA:** | |
|---|---|
| Reaktionsmix | 50µl |
| Binding-buffer | 250 µl |
| Carrier (cot-1-DNA, 100ng/µl) | 3 µl |

Den Mix auf die Säulchen pipettieren und für 1 Minute in einer Tischzentrifuge bei max. Umdrehung zentrifugieren. Den Durchlauf verwerfen, die Säulchen mit 500 µl Waschpuffer auffüllen und wie oben zentrifugieren. Den Durchlauf verwerfen, die Säulchen mit 200 µl Waschpuffer auffüllen und wie oben zentrifugieren.

Die Säulchen in ein neues 1,5 ml-Eppendorf Gefäß überführen und mit 50 µl Elutions-Puffer auffüllen, eine Minute inkubieren und dann wie oben zentrifugieren. Den Elutionsvorgang einmal wie beschrieben wiederholen.

### B5. Ethanol-Fällung der gereinigten cDNA

Die Pellet Paint^{™}-Carrier-Stocklösung nicht vortexen und immer dunkel aufbewahren. Längere Lagerung bei -20°C, kleinere Aliquots können für ca. 1 Monat bei 4°C gelagert werden.

| **Ethanol Fällung:** | |
|---|---|
| Eluat | 100 µl |
| Carrier (Pellet Paint^{™}) | 2 µl |
| Natrium-Acetat | 10 µl |
| Alkohol abs | 220 µl |

Den Ansatz gut mischen (nicht vortexen) und die cDNA bei Raumtemperatur für 10 Minuten bei maximaler Umdrehung pelletieren. Den Überstand abnehmen und das Pellet einmal mit 200 µl 70% Ethanol waschen. 1 Minute wie oben zentrifugieren und den Überstand **vollständig** mit einer Pipette entfernen. Zum Trocknen des Pellets das Eppendorf-Gefäß für ca. 5 Minuten geöffnet bei Raumtemperatur stehen lassen. **Nicht in der speedvac trocknen!** Anschließend das Pellet in 8 µl Tns-Puffer (pH 8,5) lösen und auf Eis stellen.

### B6. Zweite Amplifikation per in vitro-Transkription

| **In vitro Transkription:** | |
|---|---|
| Template DANN | 8 µl |
| ATP/CTP/GTP/UTP (je 75 mM) | 2 µl |
| 10x Puffer | 2 µl |
| T7-RNA-Polymerase | 2 µl |

Alle Reaktionskomponenten auftauen und den Ansatz **bei Raumtemperatur, niemals auf Eis** zusammenpipettieren, da der Spermidin-Anteil im Reaktionspuffer zu einer Präzipitation des Templates führen würde. Für die Reaktion 0,5 ml bzw. 0,2 ml Rnase-frei PCR-Tubes verwenden.

Die Transkription über Nacht bei 37°C in einem Thermocycler mit Heizdeckel oder in einem Hybridisierungsofen inkubieren. 1-2 µl des Ansatzes auf ein natives 1,5%-Agarose-Gel auftragen. Anschließend 1 µl DNase pro Reaktion zugeben und für weitere 15 Minuten bei 37°C inkubieren. Zur Reinigung der RNA das Rneasy-Kit von Qiagen benutzen und nach dem Protokoll zur RNA-clean-up vorgeben. Die RNA anschließend mit 2x50 µl DEPC-Wasser eluieren und wie bei Schritt 6 beschrieben mit Ethanol fällen. Das RNA-Pellet in 5 µl DEPC-Wasser lösen.

Die RNA ist jetzt fertig um eine Markierung für die Microarray-Hybridisierung, bzw. um eine weitere Amplifikationsrunde durchzuführen (diese erfolgt exakt wie in B1-B6 beschrieben).

## Patentansprüche

1. Verfahren zur Vermehrung von Ribonukleinsäuren, Schritte umfassend, bei denen man
(a) mittels eines Einzelstrang-Primers definierter Sequenz, einer RNA-abhängigen DNA-Polymerase und Desoxyribonukleosidtriphosphaten einen DNA-Einzelstrang durch reverse Transkription aus einer RNA erzeugt;
(b) die RNA entfernt;
(c) mittels eines Einzelstrang-Primers, einer DNA-Polymerase und Desoxyribonukleosidtriphosphaten einen DNA-Doppelstrang erzeugt, wobei der Einzelstrang-Primer eine Box-Sequenz, eine Sequenz von 3 bis 6 beliebigen Nukleotiden sowie eine ausgewählte Trinukleotidsequenz umfaßt, wobei sich die Box-Sequenz im 5'-Bereich des Einzelstrang-Primers befindet und eine Sequenz mit einer Länge von 10-25 Nukleotiden umfaßt, welche eine geringe Homologie zu Gensequenzen aufweist, welche von den Organismen exprimiert werden, aus denen die zu vermehrende RNA gewonnen wurde;
(d) den Doppelstrang in Einzelstränge auftrennt;
(e) mittels eines Einzelstrang-Primers, der im 5'-Bereich die Sequenz eines Promotors und im 3'-Bereich dieselbe definierte Sequenz wie der Primer in (a) umfaßt, einer DNA-Polymerase und Desoxyribonukleosidtriphosphaten DNA-Doppelstränge aus einem der in (d) entstandenen Einzelsträngen erzeugt;
(f) mittels einer RNA-Polymerase und Ribonukleosidtriphosphaten eine Vielzahl von RNA-Einzelsträngen erzeugt, die an beiden Enden definierte Sequenzen umfassen.

2. Verfahren gemäß Anspruch 1, bei dem RNA-Einzelstränge die inverse Sinnrichtung (antisense Sequenz) wie das RNA-Ausgangsmaterial aufweisen.

3. Verfahren gemäß Anspruch 1 oder 2, bei dem der in (a) verwendete Einzelstrang-Primer eine Oligo-dT-Sequenz umfaßt.

4. Verfahren gemäß einem der Ansprüche 1-3, bei dem man in (a) einen 5'-(dT)₁₈V-Primer für die reverse Transkription verwendet, worin V ein Desoxyribonukleotid-Monomer bezeichnet, das nicht dT ist.

5. Verfahren gemäß einem der vorstehenden Ansprüche, bei dem man die RNA in (b) durch RNase hydrolysiert.

6. Verfahren gemäß einem der vorstehenden Ansprüche, bei dem man die RNA in (b) durch RNase I und/oder RNase H entfernt.

7. Verfahren gemäß einem der vorstehenden Ansprüche, bei dem man in (c) einen Einzelstrang-Primer folgender Sequenz GCA TCA TAC AAG CTT GGT ACC NNN NNN TCT (30 nt) verwendet.

8. Verfahren gemäß einem der vorstehenden Ansprüche, bei dem man als DNA-Polymerase eine Reverse Transkriptase verwendet.

9. Verfahren gemäß einem der vorstehenden Ansprüche, bei dem man als Desoxyribonukleosidtriphosphate dATP, dCTP, dGTP und dTTP einsetzt.

10. Verfahren gemäß einem der vorstehenden Ansprüche, bei dem man die DNA-Doppelstränge in (d) durch Hitzeeinwirkung in Einzelstränge auftrennt.

11. Verfahren gemäß einem der vorstehenden Ansprüche, bei dem der in (e) eingesetzte Einzelstrang-Primer die Sequenz des T7-, T3- oder S6-RNA-Polymerase-Promotors umfaßt.

12. Verfahren gemäß einem der vorstehenden Ansprüche, bei dem man in (e) einen Einzelstrang-Primer verwendet, der neben der Sequenz des Promotors eine Oligo-(dT)-Sequenz von mindestens 8 Nukleotiden umfaßt.

13. Verfahren gemäß einem der vorstehenden Ansprüche, bei dem man in (e) einen Einzelstrang-Primer folgender Sequenz ACT AAT ACg ACT CAC TAT A g⁺¹ g (dT)₁₈V (40 nt) verwendet.

14. Verfahren gemäß einem der vorstehenden Ansprüche, bei dem die in (f) eingesetzte RNA-Polymerase die T7-RNA-Polymerase ist.

15. Verfahren gemäß einem der vorstehenden Ansprüche, bei dem man als Ribonukleosidtriphosphate ATP, CTP, GTP und UTP einsetzt.

16. Verfahren gemäß einem der vorstehenden Ansprüche, bei dem eine Vermehrung der Ausgangs-RNA-Sequenz um einen Faktor von mindestens 500, vorzugsweise mehr als 1000 erfolgt.

17. Verfahren gemäß einem der vorstehenden Ansprüche, bei dem man im Anschluß an (f) die folgenden Schritte zur weiteren Vermehrung der Ribonukleinsäuren durchführt:
(g) aus den in (f) erzeugten RNA-Einzelsträngen, einem Einzelstrang-Primer, der die Box-Sequenz umfaßt, einer RNA-abhängigen DNA-Polymerase und Desoxyribonukleosidtriphosphaten wird ein DNA-Einzelstrang durch reverse Transkription erzeugt;
(h) die RNA wird entfernt;
(i) aus den in (h) erzeugten DNA-Einzelsträngen werden mittels eines Einzelstrang-Primers, der im 5'-Bereich die Sequenz eines Promotors und im 3'-Bereich dieselbe definierte Sequenz, wie der in (a) genutzte Primer umfaßt, einer DNA-Polymerase und Desoxyribonukleosidtriphosphaten DNA-Doppelstränge erzeugt; und
(j) mittels einer RNA-Polymerase und Ribonukleosidtriphosphaten eine Vielzahl von RNA-Einzelsträngen erzeugt.

18. Verfahren gemäß Anspruch 17, bei dem der in (i) genutzte Einzelstrang-Primer dieselbe Sequenz wie der in (e) genutzte Primer aufweist.

19. Verfahren gemäß Anspruch 17 oder 18, bei dem die RNA in (h) mittels einer RNase hydrolysiert wird.

20. Verfahren gemäß einem der Ansprüche 17 bis 19, bei dem alle in (j) erzeugten RNA-Einzelstränge inverse Sinnrichtung aufweisen.

21. Kit zur Vermehrung von Ribonukleinsäuren gemäß einem Verfahren der Ansprüche 1 bis 20, welches die folgenden Bestandteile umfaßt:
(a) mindestens einen Einzelstrang-Primer, der die Sequenz eines Promotors umfaßt;
(b) mindestens einen Einzelstrang-Primer, wobei der Einzelstrang-Primer eine Box-Sequenz, eine Sequenz von 3 bis 6 beliebigen Nukleotiden sowie eine ausgewählte Trinukleotidsequenz umfaßt, wobei sich die Box-Sequenz im 5'-Bereich des Einzelstrang-Primers befindet und eine Sequenz mit einer Länge von 10-25 Nukleotiden umfaßt, welche eine geringe Homologie zu Gensequenzen aufweist, welche von den Organismen exprimiert werden, aus denen die zu vermehrende RNA gewonnen wurde;
(c) eine RNA-abhängige DNA-Polymerase;
(d) Desoxyribonukleosidtriphosphate;
(e) eine DNA-abhängige DNA-Polymerase;
(f) eine RNA-Polymerase; und
(g) Ribonukleosidtriphosphate.

22. Kit gemäß Anspruch 21, das drei verschiedene Einzelstrang-Primer umfaßt.

23. Kit gemäß Anspruch 21, in dem der Einzelstrang-Primer, der die Promotor-Sequenz umfaßt, auch eine Oligo-dT-Sequenz umfaßt.

24. Kit gemäß Anspruch 21, in dem ein Einzelstrang-Primer eine 5'-(dT)₁₈V-Primer-Sequenz umfaßt, worin V ein Desoxyribonukleotid-Monomer bezeichnet, das nicht dT ist.

25. Kit gemäß einem der Ansprüche 21 bis 24, das zusätzlich RNase I und/oder RNase H umfaßt.

26. Kit gemäß einem der Ansprüche 21 bis 25, in dem ein Einzelstrang-Primer die Sequenz des T7- oder S6-RNA-Polymerase-Promotors umfaßt.

27. Kit gemäß einem der Ansprüche 21 bis 26, das einen Einzelstrang-Primer folgender Sequenz ACT AAT ACg ACT CAC TAT A g⁺¹ g (dT)₁₈V (40 nt) umfaßt.

28. Kit gemäß einem der Ansprüche 21 bis 27, welches die Reverse Transkriptase als DNA-Polymerase umfaßt.

29. Kit gemäß einem der Ansprüche 21 bis 28, das die T7-RNA-Polymerase umfaßt.

30. Kit gemäß einem der Ansprüche 21 bis 29, das eine Zusammenstellung zur Markierung von DNA mit einer nachweisbaren Verbindung umfaßt.

31. Kit gemäß einem der Ansprüche 21 bis 30, das einen DNA-Microarray umfaßt.

32. Verfahren zur Analyse von Nukleinsäuren, bei dem man eine Ribonukleinsäure gewinnt, mittels eines Verfahrens gemäß einem der Ansprüche 1 bis 20 vermehrt und unter Verwendung eines Microarrays analysiert.

33. Verfahren gemäß Anspruch 32, bei dem man die Ribonukleinsäure durch Isolierung aus einer biologischen Probe gewinnt.

34. Verfahren gemäß Anspruch 32 oder 33, bei dem man die Ribonukleinsäure vermehrt, durch reverse Transkription in cDNA überführt und die cDNA mittels Microarrays analysiert.

35. Verfahren gemäß einem der Ansprüche 32 bis 34, bei dem man die Menge und/oder Sequenz der cDNA analysiert.

## Claims

1. Process for the amplification of ribonucleic acids comprising the following steps:
a) a single stranded DNA is produced from an RNA by means of reverse transcription, using a single-stranded primer having a defined sequence, an RNA-dependent DNA polymerase and deoxyribonucleoside triphosphates;
b) the RNA is removed;
c) a DNA duplex is produced by means of a single-stranded primer, a DNA polymerase and deoxyribonucleoside triphosphates, wherein the a single-stranded primer comprises a box sequence and a sequence of 3 to 6 oligonucleotides of random sequence, wherein the Box sequence is present in the 5' region of the single stranded primer and comprises a sequence with a length of 10 to 25 nucleotides having a low homology to sequences expressed by organisms from which the RNA to be amplified was obtained;
d) the duplex is separated into single-stranded DNAs;
e) DNA duplexes are produced from one of the single-stranded DNAs obtained in step (d) by means of a single-stranded primer comprising a promoter sequence at its 5'end and the same defined sequence as the primer used in step (a) at its 3'end , a DNA polymerase and deoxyribonucleoside triphosphates;
f) a plurality of single stranded RNAs is produced, both ends of which comprise defined sequences, by means of an RNA polymerase and ribonucleoside triphosphates.

2. Process according to claim 1, wherein the single-stranded RNA obtained have antisense orientation (antisense sequence) in relation to the RNA starting material.

3. Process according to claims 1 and 2, **characterized in that** the single-stranded primer used in step (a) comprises an oligo-dT-sequence.

4. Process according to claims 1-3, **characterized in that** a 5'-(dT)₁₈V-primer is used in step (a) for reverse transcription, with V being any deoxyribonucleotide-monomer other than dT.

5. Process according to any of the claims above, **characterized in that** the RNA is hydrolyzed by means of RNase in step (b).

6. Process according to any of the claims above, **characterized in that** the RNA is removed by means of RNase I and/or RNase H in step (b).

7. Process according to any of the claims above, **characterized in that** a single-stranded primer is used with the following sequence: GCA TCA TAC AAG CTT GGT ACC NNN NNN TCT (30 nt) in step (c).

8. Process according to any of the claims above, **characterized in that** a reverse transcriptase is used as DNA polymerase.

9. Process according to any of the claims above, **characterized in that** dATP, dCTP, dGTP and dTTP are used as deoxyribonucleotide-monomers.

10. Process according to any of the claims above, **characterized in that** DNA double strands are separated in single strands by means of heat in step (d).

11. Process according to any of the claims above, **characterized in that** a single-stranded primer is used, which comprises the sequence of either the T7, T3 or SP6 RNA polymerase in step (e).

12. Process according to any of the claims above, **characterized in that** a single-stranded primer is used, containing not in addition to promoter sequence an oligo(dT)-sequence of at least 8 nucleotides in step (e).

13. Process according to any of the claims above, **characterized in that** the single-stranded primer used in step (e)has the following sequence: ACT AAT ACg ACT CAC TAT A g⁺¹ g (dT)₁₈V (40 nt).

14. Process according to any of the claims above, **characterized in that** T7 RNA polymerase is used as RNA polymerase in step (f).

15. Process according to any of the claims above, **characterized in that** ATP, CTP, GTP and UTP are used as ribonucleotide-monomers.

16. Process according to any of the claims above, **characterized in that** the amplification factor of the starting RNA sequence is at least 500, preferably more than 1000.

17. Process according to any of the claims above, **characterized in that** the method comprises after step (f) the following steps for further amplification of ribonucleic acids:
(g) single-stranded DNA is synthesized using the single-stranded RNAs generated in step (f) as template, reverse transcriptase, a single-stranded primer containing the Box sequence, an RNA-dependant DNA polymerase and deoxyribonucleoside triphosphates;
(e) the RNA is removed;
(f) double-stranded DNA is synthesized using the single-stranded DNA generated in (h) as a template, a single-stranded primer comprising a promoter sequence in its 5' region and the same defined sequence as the primer used in step (a), in its 3' region, a DNA polymerase and deoxyribonucleoside triphosphates;
(h) a multitude of single-stranded RNAs is synthesized using an RNA polymerase and ribonucleoside triphosphates.

18. Process according to any of the claims above, **characterized in that** the single-stranded primer used in step (i) is identical with the single-stranded primer used in step (e).

19. Process according to any of the claims above, **characterized in that** the RNA is hydrolyzed by means of RNase in step (h).

20. Process according to claims above, **characterized in that** all single-stranded RNAs produced in step (j) have antisense sequence.

21. Kit for ribonucleic acid amplification according to claims 1 to 20, comprising the following components:
a) at least one single-stranded primer comprising a promoter sequence;
b) at least one single-stranded primer comprising a box sequence, a sequence of 3 to 6 oligonucleotides of random sequence and a specific trinucleotide seqeunce, wherein the Box sequence is present in the 5' region of the single stranded primer and comprises a sequence with a length of 10 to 25 nucleotides having a low homology to sequences expressed by organisms from which the RNA to be amplified was obtained;
c) an RNA-dependent DNA polymerase;
d) deoxyribonucleoside triphosphates;
e) a DNA-dependent DNA polymerase;
f) an RNA polymerase; and
g) ribonucleoside triphosphates.

22. Kit according to claim 21, **characterized in that** the kit comprises three different single-stranded primers.

23. Kit according to claim 21, **characterized in that** the single-stranded primer which comprises the promoter sequence, further comprises an oligo-dT-sequence.

24. Kit according to claim 21, **characterized in that** a single-stranded primer comprises a 5'-(dT)₁₈V-primer sequence, wherein V is any deoxyribonucleotide-monomer other than dT.

25. Kit according to claim 21 to 24, **characterized in that** the kit further comprises RNase I and/or RNase H.

26. Kit according to claim 21 to 25, **characterized in that** the kit comprises a single-stranded primer with a T7, T3 or SP6 RNA polymerase promoter sequence.

27. Kit according to claim 21 to 26, **characterized in that** the single-stranded primer comprises the following sequence: ACT AAT ACg ACT CAC TAT A g⁺¹ g (dT)₁₈V (40 nt).

28. Kit according to claim 21 to 27, **characterized in that** it comprises a reverse transcriptase as a DNA polymerase.

29. Kit according to claim 21 to 28, **characterized in that** it comprises the T7 RNA polymerase.

30. Kit according to claim 21 to 29, **characterized in that** it comprises a composition for labeling of DNA with a detectable moiety.

31. Kit according to claim 21 to 30, **characterized in that** the kit includes a DNA-microarray.

32. Process for nucleic acid analysis that involves production of ribonucleic acids, amplification with a process according to one of claims 1 to 20, and analysis by means of microarrays.

33. Process according to claim 32 **characterized in that** the ribonucleic acids are isolated from a biological sample.

34. Process according to claims 32 or 33, **characterized in that** ribonucleic acids are amplified, converted to cDNA by means of reverse transcription, and the cDNAs are analyzed by means of micoarrays.

35. Process according to claims 32 to 34, **characterized in that** the amount and/or sequence of the cDNA are analyzed.

## Revendications

1. Procédé d'amplification d'acides ribonucléiques, comprenant les étapes, dans lesquelles :
(a) à l'aide d'une amorce simple brin de séquence définie, d'une ADN polymérase dépendant de l'ARN et de désoxyribonucléoside-triphosphates, on produit un ADN simple brin par transcription inverse à partir d'un ARN ;
(b) on élimine l'ARN ;
(c) à l'aide d'une amorce simple brin, d'une ADN polymérase et de désoxyribonucléosides-triphosphates, on produit un ADN double brin, dans lequel l'amorce simple brin comprend une séquence de type boîte, une séquence de 3 à 6 nucléotides quelconques, ainsi qu'une séquence trinucléotidique choisie, la séquence de type boîte se situant dans la région en 5' de l'amorce simple brin et comprenant une séquence d'une longueur de 10 à 25 nucléotides qui présente une faible homologie avec des séquences de gènes qui sont exprimées par les organismes à partir desquels provient l'ARN que l'on souhaite amplifier ;
(d) on sépare le double brin en simples brins ;
(e) à l'aide d'une amorce simple brin, qui comprend dans la région en 5' la séquence d'un promoteur et, dans la région en 3', la même séquence définie que celle de l'amorce décrite à l'étape (a), d'une ADN polymérase et de désoxyribonucléosides-triphosphates, on produit des ADN doubles brins à partir de l'un des simples brins produits à l'étape (d) ; et
(f) à l'aide d'une ARN polymérase et de ribonucléosides-triphosphates, on produit une pluralité d'ARN simples brins, qui comprennent des séquences définies aux deux extrémités.

2. Procédé selon la revendication 1, dans lequel les ARN simples brins présentent le sens inverse (séquence antisens) à celui du matériau d'ARN de départ.

3. Procédé selon la revendication 1 ou 2, dans lequel l'amorce simple brin utilisée à l'étape (a) comprend une séquence de type oligo-dT.

4. Procédé selon l'une quelconque des revendications 1 à 3, dans lequel on utilise à l'étape (a) une amorce de type 5'-(dT)₁₈V pour la transcription inverse, dans laquelle le terme V désigne un monomère de désoxyribonucléotide, qui ne représente pas dT.

5. Procédé selon l'une quelconque des revendications précédentes, dans lequel on hydrolyse l'ARN à l'étape (b) au moyen d'une RNase.

6. Procédé selon l'une quelconque des revendications précédentes, dans lequel on élimine l'ARN à l'étape (b) en utilisant la RNase 1 et/ou la RNase H.

7. Procédé selon l'une quelconque des revendications précédentes, dans lequel on utilise à l'étape (c) une amorce simple brin de séquence suivante GCA TCA TAC AAG CTT GGT ACC NNN NNN TCT (30 nt).

8. Procédé selon l'une quelconque des revendications précédentes, dans lequel on utilise, comme ADN polymérase, une transcriptase inverse.

9. Procédé selon l'une quelconque des revendications précédentes, dans lequel on utilise, comme désoxyribonucléosides-triphosphates, le dATP, le dCTP, le dGTP et le dTTP.

10. Procédé selon l'une quelconque des revendications précédentes, dans lequel on sépare, à l'étape (d), les ADN doubles brins en simples brins par action de la chaleur.

11. Procédé selon l'une quelconque des revendications précédentes, dans lequel l'amorce simple brin utilisée à l'étape (e) comprend la séquence de promoteur de l'ARN polymérase de T7, de T3 ou de S6.

12. Procédé selon l'une quelconque des revendications précédentes, dans lequel on utilise à l'étape (e) une amorce simple brin, qui comprend, outre la séquence du promoteur, une séquence de type oligo-(dT) contenant au moins 8 nucléotides.

13. Procédé selon l'une quelconque des revendications précédentes, dans lequel on utilise à l'étape (e) une amorce simple brin de séquence suivante ACT AAT ACg ACT CAC TAT A g⁺¹ g (dT)₁₈V (40 nt).

14. Procédé selon l'une quelconque des revendications précédentes, dans lequel l'ARN polymérase utilisée à l'étape (f) est l'ARN polymérase de T7.

15. Procédé selon l'une quelconque des revendications précédentes, dans lequel on utilise, comme ribonucléosides-triphosphates, l'ATP, le CTP, le GTP et l'UTP.

16. Procédé selon l'une quelconque des revendications précédentes, dans lequel on obtient une amplification de la séquence d'ARN de départ d'un facteur d'au moins 500, de préférence, de plus de 1000.

17. Procédé selon l'une quelconque des revendications précédentes, dans lequel on effectue, à la suite de l'étape (f), les étapes suivantes pour amplifier encore les acides ribonucléiques :
(g) à partir des ARN simples brins produits à l'étape (f), d'une amorce simple brin qui comprend la séquence de type boîte, d'une ADN polymérase dépendant de l'ARN et de désoxyribonucléosides-triphosphates, on produit un ADN simple brin par transcription inverse ;
(h) on élimine l'ARN ;
(i) à partir des ADN simples brins produits à l'étape (h), à l'aide d'une amorce simple brin, qui comprend dans la région en 5' la séquence d'un promoteur et dans la région en 3' la même séquence définie que celle de l'amorce utilisée à l'étape (a), d'une ADN polymérase et de désoxyribonucléosides-triphosphates, on produit des ADN doubles brins ; et
(j) à l'aide d'une ARN polymérase et de ribonucléosides-triphosphates, on produit une pluralité d'ARN simples brins.

18. Procédé selon la revendication 17, dans lequel l'amorce simple brin utilisée à l'étape (i) présente la même séquence que l'amorce utilisée à l'étape (e).

19. Procédé selon la revendication 17 ou 18, dans lequel l'ARN obtenu à l'étape (h) est hydrolyse à l'aide d'une RNase.

20. Procédé selon l'une quelconque des revendications 17 à 19, dans lequel tous les ARN simples brins produits à l'étape (j) présentent un sens inverse.

21. Trousse destinée à l'amplification d'acides ribonucléiques selon un procédé des revendications 1 à 20, qui comprend les composants suivants :
(a) au moins une amorce simple brin, qui comprend la séquence d'un promoteur ;
(b) au moins une amorce simple brin, laquelle amorce simple brin comprenant une séquence de type boîte, une séquence de 3 à 6 nucléotides quelconques, ainsi qu'une séquence trinucléotidique choisie, dans laquelle la séquence de type boîte se situe dans la région en 5' de l'amorce simple brin, et une séquence d'une longueur de 10 à 25 nucléotides, qui présente une faible homologie avec des séquences de gènes qui sont exprimées par les organismes à partir desquels provient l'ARN que l'on souhaite amplifier ;
(c) une ADN polymérase qui dépend de l'ARN ;
(d) des désoxyribonucléoside-triphosphates ;
(e) une ADN polymérase qui dépend de l'ADN ;
(f) une ARN polymérase ; et
(g) des ribonucléosides-triphosphates.

22. Trousse selon la revendication 21, qui comprend trois différentes amorces simples brins.

23. Trousse selon la revendication 21, dans laquelle l'amorce simple brin, qui comprend la séquence de promoteur, comprend également une séquence oligo-dT.

24. Trousse selon la revendication 21, dans laquelle une amorce simple brin comprend une séquence d'amorce de type 5'-(dT)₁₈V, dans laquelle le terme V désigne un monomère de désoxyribonucléotide qui ne représente pas dT.

25. Trousse selon l'une quelconque des revendications 21 à 24, qui comprend en plus de la RNase I et/ou de la RNase H.

26. Trousse selon l'une quelconque des revendications 21 à 25, dans laquelle une amorce simple brin comprend la séquence du promoteur de l'ARN polymérase de T7 ou de S6.

27. Trousse selon l'une quelconque des revendications 21 à 26, qui comprend une amorce simple brin de séquence suivante ACT AAT ACg ACT CAC TAT A g⁺¹ g (dT)₁₈V (40 nt).

28. Trousse selon l'une quelconque des revendications 21 à 27, qui comprend la transcriptase inverse comme ADN polymérase.

29. Trousse selon l'une quelconque des revendications 21 à 28, qui comprend l'ARN polymérase T7.

30. Trousse selon l'une quelconque des revendications 21 à 29, qui comprend une composition pour le marquage de l'ADN avec un composé de détection.

31. Trousse selon l'une quelconque des revendications 21 à 30, qui comprend un microréseau d'ADN.

32. Procédé d'analyse d'acides nucléiques, dans lequel on obtient un acide ribonucléique, on amplifie ce dernier au moyen d'un procédé selon l'une quelconque des revendications 1 à 20 et on l'analyse en utilisant un microréseau.

33. Procédé selon la revendication 32, dans lequel l'acide ribonucléique est obtenu par isolement à partir d'un échantillon biologique.

34. Procédé selon la revendi cation 32 ou 33, dans lequel on amplifie l'acide ribonucléique, on le convertit sous forme d'ADNc par transcription inverse et on analyse l'ADNc au moyen d'un microréseau.

35. Procédé selon l'une quelconque des revendications 32 à 34, dans lequel on analyse la quantité et/ou la séquence de l'ADNc.
